(19) Europäisches Patentamt
European Patent Office
Office européen des brevets

(11) Publication number: 0 027 679
B1

(12) **EUROPEAN PATENT SPECIFICATION**

(45) Date of publication of patent specification: 10.08.83

(21) Application number: 80302207.8

(22) Date of filing: 01.07.80

(51) Int. Cl.³: C 07 D 215/44, C 07 D 215/60, A 61 K 31/47 //C07C91/44, C07C79/26

# (54) Substituted-5-((7-chloro-4-quinolinyl)amino)-3-(aminomethyl)-(1,1'-biphenyl)-2-ol compounds; processes for their production; and pharmaceutical compositions containing the compounds.

(30) Priority: 18.10.79 US 86226

(43) Date of publication of application: 29.04.81 Bulletin 81/17

(45) Publication of the grant of the patent: 10.08.83 Bulletin 83/32

(84) Designated Contracting States: AT BE CH DE FR GB IT LI LU NL SE

(56) References cited:
GB - A - 612 115
GB - A - 974 348
US - A - 2 474 823

CHEMICAL ABSTRACTS; vol. 89, no. 7, 14-08-1978, abstract no. 59772u, page 562 Columbus, Ohio, U.S.A.

(73) Proprietor: WARNER-LAMBERT COMPANY
201 Tabor Road
Morris Plains New Jersey 07950 (US)

(72) Inventor: Elslager, Edward Faith
4081 Thornoaks
Ann Arbor, Michigan 48104 (US)
Inventor: Werbel, Leslie Morton
1570 Covington Drive
Ann Arbor, Michigan 48104 (US)

(74) Representative: Jones, Michael Raymond et al,
Haseltine Lake & Co. 28 Southampton Buildings
Chancery Lane
London WC2A 1AT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 027 679 B1

# Substituted-5-[(7-chloro-4-quinolinyl)amino]-3-(aminomethyl)-[1,1′-biphenyl]-2-ol compounds; processes for their production; and pharmaceutical compositions containing the compounds

This invention relates to substituted-5-[(7-chloro-4-quinolinyl)amino]-3-(aminomethyl)-[1,1′-biphenyl]-2-ol compounds and salts thereof; to processes for their production; and to antimalarial pharmaceutical compositions incorporating the substituted compounds.

British Patent Specification No. 612115 discloses compounds of the following general formula:

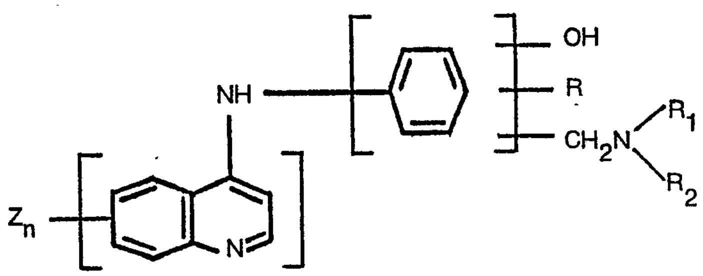

in which Z can be, inter alia, a halogen atom, $R_1$ is a hydrogen atom or alkyl radical; $R_2$ is an alkyl radical; and R is a hydrogen or halogen atom, a hydroxy group or a saturated or unsaturated alkyl radical.

One particular compound disclosed in that British specification is that in Example 1, which has the following formula:

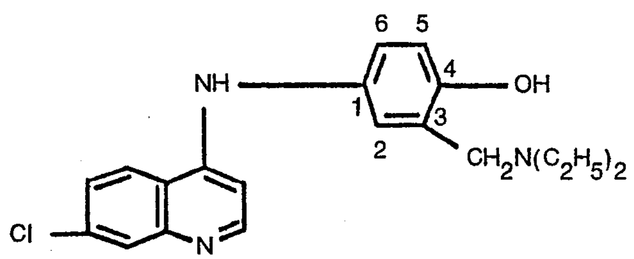

(Amodiquine)

Another particular compound disclosed in that British specification is that in Example 5, which is the 5′-allyl analogue of the compound of Example 1 and which has the following formula:

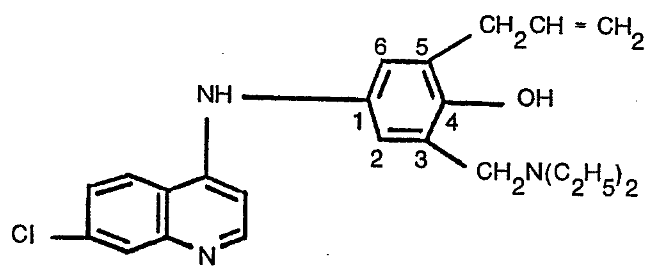

where the allyl substituent is ortho to the hydroxy group of the cresol ring.

To avoid confusion it should be noted that the numbering system of the cresol ring (i.e. that ring bearing the OH group) in British Patent Specification No. 612115 is *different* from that used herein in respect of the compounds of the present invention.

An article by J. H. Burckhalter *et al* in the Journal of the American Chemical Society 70, (1948), 1363—1373, hereinafter referred to as the Burckhalter article, discloses the amodiquine compound of Example 1 of British Patent Specification No. 612115 as compound 9 in Table VI (compound SN—10751) on page 1366, and shows it to have a quinine equivalent of 25.

The chloro analogue, in which the chloro is ortho to the OH of the cresol ring and which has the formula:

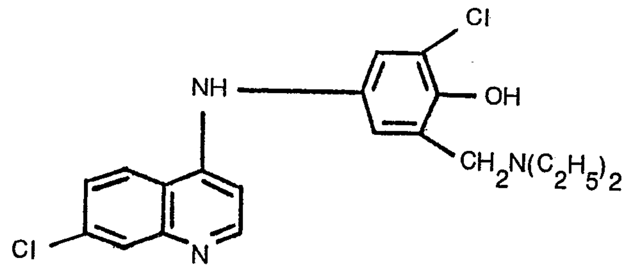

is disclosed as compound 4 in Table XI (compound SN—13729) on page 1369 of the Burckhalter article. The presence of the additional chlorine substituent in a position ortho to the hydroxy group of the cresol ring causes the quinine equivalent to *fall* from 25 to 12.

Replacement of the chlorine substituent (ortho to the hydroxy group) by an allyl substituent (the compound of Example 5 of British Patent Specification No. 612115) is disclosed in the Burckhalter article, as compound 7 in Table XI (compound SN—11991). This particular substitution at the position ortho to the hydroxy group of the cresol ring causes the quinine equivalent to *fall* even *further,* in fact to 10.

It can thus be appreciated that the prior art shows that substitution at the position ortho to the hydroxy group of the cresol ring leads to a fall in the value of the quinine equivalent and hence directs attention *away* from further research into substitution at that position. This much is clear from compound 5 in Table XI on page 1369 of the Burckhalter article, which has the formula:

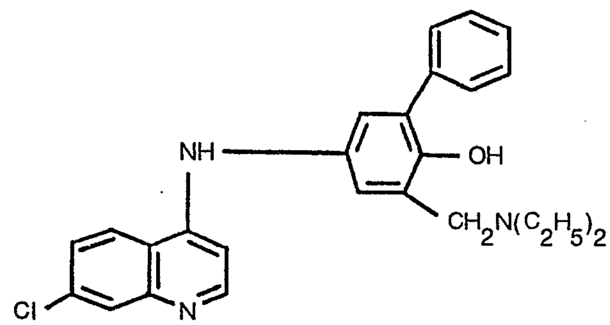

This particular compound, which has an *un*substituted phenyl group at the position ortho to the hydroxy group, was not even made in a quantity sufficient for testing the quinine equivalent, thus demonstrating the researchers' lack of faith in the efficacy of a compound substituted at that position.

Contrary to the reasonable expectations derived from the prior art, the compounds of the present invention having a *substituted* phenyl group at the position ortho to the hydroxy group of the cresol ring display good anti-malarial activity, in fact the ability to treat resistant forms of malaria, as is evidenced by some of the data provided later herein which show that certain of the compounds of the present invention have twenty times the activity of the known compound amodiquine.

More particularly one aspect of the present invention relates to compounds of the following general formula:

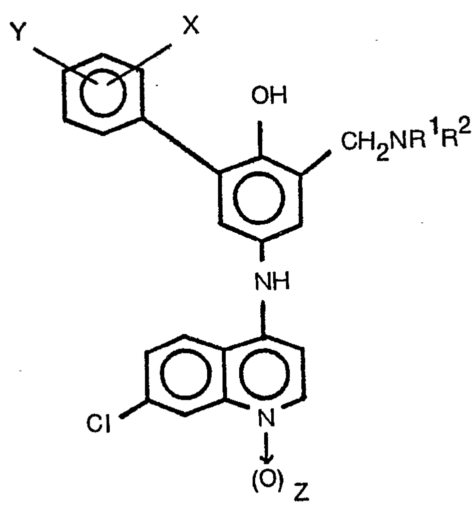

or a pharmaceutically acceptable salt thereof; wherein X is a hydrogen, fluorine, bromine or chlorine atom or a lower alkyl radical; Y is a chlorine, fluorine or bromine atom or a trifluoromethyl, lower alkoxy, cyano, hydroxy, nitro, lower alkylthio, amino, lower alkyl amino, di(lower alkyl) amino, pyrrolidino or piperidino radical; $R^1$ and $R^2$, which are the same or different, are each a hydrogen atom or a lower alkyl radical, or $R^1R^2N$ taken together is a pyrrolidino, piperidino or homopiperidino radical of which the heterocyclic ring is unsubstituted or substituted by from one to four lower alkyl radicals; Z is zero or one; a lower alkyl radical is an alkyl radical containing from one to six carbon atoms; and a lower alkoxy radical is an alkyoxy radical containing from one to six carbon atoms.

Preferably X is a hydrogen or chloro and Y is chloro or fluoro. Also, preferably X is in the 3-position and Y is in the two or four position, $R_1$ is lower alkyl, $R_2$ is hydrogen or lower alkyl and Z is zero or one.

The term "lower alkyl" is intended to mean a hydrocarbon fragment of from one to six carbon atoms.

The term "lower alkoxy" is intended to mean a lower alkyl group linked directly to an oxygen atom by a single bond.

The term "pharmaceutically acceptable salts" is intended to mean the relatively non-toxic acid-addition salts, such as the hydrochloride sulfate, phosphate, acetate, benzoate methane sulfonate, salt or the salt formed with a base, such as the sodium, potassium or ammonium salt. In addition, of special interest are typical repository salts, such as the pamoate.

The compounds of the invention can be prepared by coupling a compound of the formula

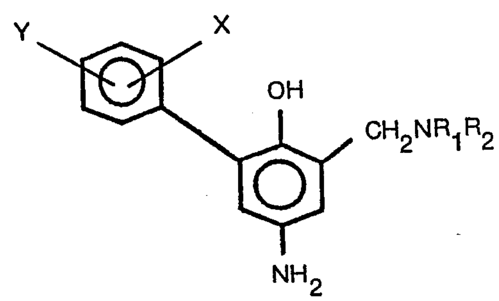

II.

with a compound of the formula

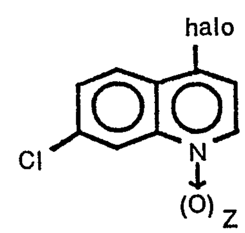

III.

wherein $R_1$, $R_2$, X, Y and Z are as previously defined and halo is iodo, chloro or bromo.

Approximately equimolar quantities of reactants are employed in a polar solvent, such as ethanol, dimethylformamide, dimethylsulfoxide, acetonitrile, or mixture thereof. The reaction requires the presence of an acid, such as hydrochloric or benzenesulfonic, in at least catalytic amounts to quantities on a molar basis in excess of the reactants.

The reaction is carried out for periods of about one-half hour to twelve hours, preferably one to five hours, at a temperature range of about 10°C to 150°C, preferably 35°C to 80°C.

While the starting material of the formula III is known, compounds of the formula II are prepared by removing an acyl group from a compound of the formula

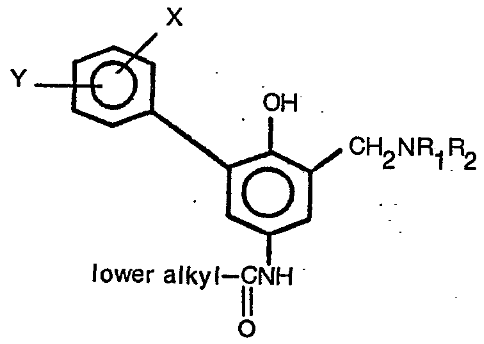

IV.

where X, Y, $R_1$ and $R_2$ are as previously defined.

The term "homopiperidino" is used herein to mean a homologue of a piperidino radical having an extra methylene group in the ring, that is a seven membered ring containing one nitrogen and six carbon atoms.

The intermediates of the formula IV are prepared by reacting a compound of the formula

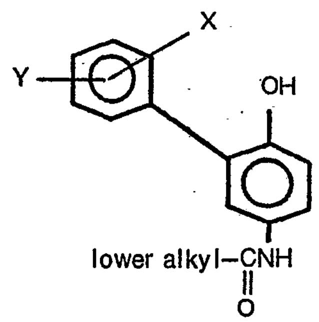

V.

with formaldehyde and an amine of the formula

$$HNR_1R_2 \cdot HCl$$

where X, Y, $R_1$ and $R_2$ are as previously defined.

The intermediates of the formula V are prepared by acylating a compound of the formula

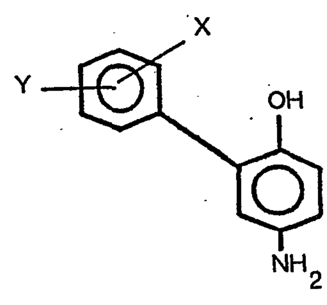

VI.

using a lower alkyl acyl halide or anhydride.

The intermediates of the formula VI may be prepared by two routes, the much preferred procedure, which is a part of this invention, utilizes the reduction of a compound of the formula

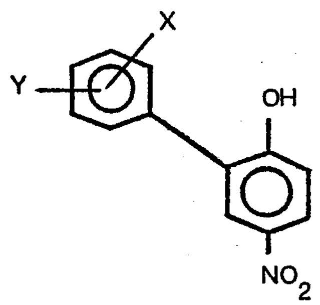

VII.

wherein X and Y are as previously defined.

The reduction can preferably be carried out catalytically using finely divided Ni, Pd, Pt, etc., which may or may not be on a support such as carbon or alumina, and hydrogen in a polar solvent, such as ethanol or acetic acid. The reaction requires from only about a few minutes to about six hours at from 10°C to 60°C.

The intermediates of the formula VII are obtained by reacting a compound of the formula

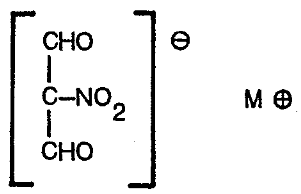

VIII.

wherein M⊕ is a cation, preferably sodium or potassium, with a compound of the formula

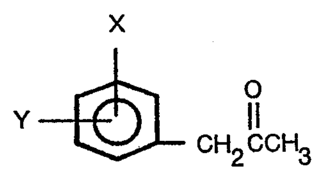

IX.

wherein X and Y are as previously defined, especially when X is hydrogen and Y is in the para position.

Approximately equimolar quantities of reactants are employed in a polar solvent, such as water, ethanol, dimethylformamide, dimethylsulfoxide, or mixture thereof. The reaction requires the presence of a base such as sodium hydroxide or potassium hydroxide, in at least an equimolar ratio when compared to reactants, preferably on a two to one basis in favor of the base.

The reaction is carried out for period of from two to twenty-four hours at a temperature range of 10°C to 80°C, preferably 15°C to 40°C.

The compounds of formula IX are known or easily prepared according to methods in the literature.

The second route that may be used to prepare compounds of the formula VI involves reacting a compound of the formula

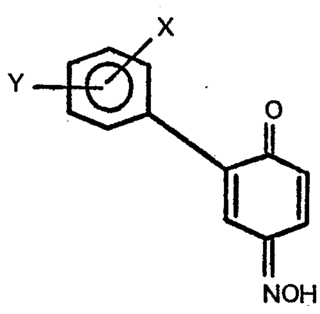

X.

with sodium dithionate.

The compounds of formula X are prepared by treating

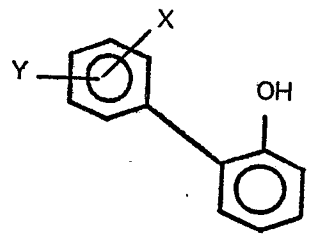

XI.

with nitrous acid or sodium nitrite in acid. The compounds of formula XI are known or easily prepared according to method in the literature.

The foregoing intermediates of the formula

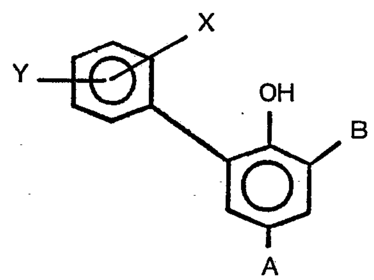

XII.

and salts thereof, wherein A is $NH_2$ or lower

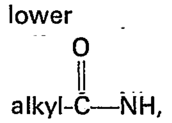

B is hydrogen or $CH_2NR_1R_2$ and X, Y, $R_1$ and $R_2$ are as previously defined with the proviso that when A is $NH_2$; B is $CH_2NR_1R_2$ are new compounds.

The compounds of formula I form salts with any of a variety of organic or inorganic acids. The preferred acids are those acids which would give rise to pharmaceutically acceptable salts, such as acetic, citric, lactic, tartaric, hydrochloric, sulfuric and phosphoric. In addition, certain of the compounds of formula I form salts with any of a variety of bases. The preferred bases are those bases which would give rise to pharmaceutically acceptable salts, such as sodium hydroxide, potassium hydroxide and calcium hydroxide.

Certain of the compounds of the invention may exist in the form of hydrates or solvates. These forms are considered to be equivalent to the compounds of the invention and encompassed by formula I and its salts.

The compounds of the invention are new chemical compounds that are useful as pharmacological agents. The compounds are potent antimalarial agents. The compounds exhibit the highly desirable property of not only exhibiting a marked activity against normal malarial parasite strains, but also against resistant strains.

The compounds of the invention may be administered orally or parenterally. Surprisingly, the compounds of the invention are also excellent repository antimalarials. It does not appear to be necessary to have a typical repository salt, such as the pamoate salt, to achieve a repository effect, although the effect may be enhanced by the use of such a salt.

When administered orally or parenterally, the dose is adjusted to the condition of the individual patient; however, the usual mammalian daily dose range is from about 0.1 mg/kg of body weight to about 25 mg/kg of body weight, preferably 0.5 mg/kg to 5.0 mg/kg. Thus, a 70 kg mammal would have administered about 7.0 mg to about 1.8 g over a twenty-four hour period.

The above employed pharmaceutical compositions are produced by formulating a compound of the foregoing formula (as an active ingredient) in dosage unit form with a pharmaceutical carrier. Some examples of dosage unit forms are tablets, capsules, lozenges, and pills; as well as powders and aqueous and non-aqueous oral solutions and suspensions and parenteral solutions packaged in containers containing either one or some larger number of dosage units and capable of being subdivided into individual doses by such means as measurement into a teaspoon or other standard container. Some examples of suitable pharmaceutical carriers, including pharmaceutical diluents, are gelatin capsules; sugars such as lactose and sucrose; starches such as corn starch and potato starch; cellulose derivatives such as sodium carboxymethyl cellulose, ethyl cellulose, methyl cellulose, and cellulose acetate phthalate; gelatin; talc; stearic acid; magnesium stearate; vegetable oils such as

peanut oil, cottonseed oil, sesame oil, olive oil, corn oil, and oil of theobroma; propylene glycol, glycerine, sorbitol; polyethylene glycol; water; agar; alginic acid; isotonic saline, and phosphate buffer solutions; as well as other compatible substances normally used in pharmaceutical formulations. The composition and at least 2% in a primarily liquid composition. The most satisfactory compositions are agents, and/or preservatives. These materials, if present, are usually used in relatively small amounts. The compositions can, if desired, also contain other therapeutic agents.

The percentage of the active ingredient in the foregoing compositions can be varied within wide limits but for practical purposes it is preferably present in a concentration of at least 10% in a solid composition and at least 2% in a primarily liquid composition. The most satisfactory compositions are those in which a much higher proportion of the active ingredient is present. The compositions of the invention preferably contain from 1 to 1,000 mg of the active ingredient per dosage unit so that the entire amount to be administered during a day can be made up from a reasonable number of dosage units.

Compounds of the invention were tested against a normal drug-sensitive strain of *Plasmodium berghei* in mice by the parenteral route by a procedure described in *J. Med. Chem.,* 1043 (1967). The compounds were dissolved or suspended in sesame or peanut oil and were administered to mice in a single subcutaneous dose seventy-two hours post infection. Extension of the mean survival time of the treated mice is interpreted as evidence of anti-malarial activity. Compounds are arbitrarily considered to be "active" when they produce at least a 100 percent increase in the mean survival time of treated mice. Animals that survive to sixty days are considered "cured" (termed "C" in Tables I and II). The means survival time (MST) of infected control mice ranges from 6.1—6.3 days while the numbers in parentheses in Tables I and II indicate extension of survival time over the controls as an average for those animals of the five animal group not "cured". Table I compares a preferred compound of the invention to the widely used antimalarial, amodiaquine while Table II shows the high degree of activity of a large number of compounds of the invention.

Studies were also conducted with infections resulting from inoculations of owl monkeys with trophozoites of the chloroquine-pyrimethamine-resistant Vietnam Smith strain of *P. Falciparum*; the chloroquine-quinine-resistant, pyrimethamine-susceptible Vietnam Oak Knoll strain of *P. Falciparum*; or the pyrimethamine-resistant Uganda Palo Alto strain of *P. Falciparum.* The test method is described in L. H. Schmidt, *Trans. R. Soc. Trop. Med. Hyg. 67* 446 (1973).

The test showed that 4′-chloro-5-[(7-chloro-4-quinolinyl)amino]-3-[(diethylamino)methyl]-[1,1′-biphenyl] - 2 - ol, 1 - oxide and 4′ - chloro - 5 - [(7 - chloro - 4 - quinolinyl)amino] - 3 - [(1,1 - dimethyl - ethyl)amino]methyl]-[1,1′-biphenyl]-2-ol-1-oxide were curative against the Vietnam Smith and Vietnam Oak Knoll strains when administered orally at 2.0 mg/kg/day for a three day period and 4′-chloro-5-[(7-chloro-4-quinolinyl)amino]-3-[(diethylamino)methyl]-[1,1′-biphenyl]-2-ol and 4′-chloro-5-[(7-chloro-4-quinolinyl)amino]-3-[[(1,1-dimethylethyl)amino]methyl]-2-[1,1′-diphenyl]-2-ol cleared the parasitemia of the Vietnam Smith, Vietnam Oak Knoll and Uganda Palo Alto strains when administered orally at 1.0 mg/kg/day for three days.

By the above method, it appears that 4′-chloro-5-[(7-chloro-4-quinolinyl)amino]-3-[(diethylamino)methyl]-[1,1′-biphenyl]-2-ol-1-oxide and 4′-chloro-5-[(7-chloro-4-quinolinyl)amino]-3-[[1,1-dimethylethyl)amino]methyl]-[1,1′-biphenyl]-2-ol-1-oxide have twenty times the activity of amodiaquine as curative agents against Oak Knoll strains of the malaria parasite and that 4′-chloro-5-[(7-chloro-4-quinolinyl)amino]-3-[(diethylamino)methyl][1,1′-biphenyl]-2-ol and 4′-chloro-5-[(7-chloro-4-quinolinyl)amino]-3-[[(1,1-dimethylethyl)amino]methyl]-[1,1′-biphenyl]-2-ol have twenty times the activity of amodiaquine in clearing parasites in the above noted strain.

TABLE I

Comparison of Amodiaquine* (A) and 4′-chloro-5-[(7-chloro-4-quinolinyl)-amino]-3-[(diethylamino)methyl]-[1,1′-biphenyl]-2-ol (B) against trophozoite-induced *Plasmodium Berghei* in mice

| | MST after single s.c. dose | | | | | | |
|---|---|---|---|---|---|---|---|
| | 640 | 320 | 160 | 80 | 40 | 20 | 10 |
| A | 5C | 5C | 5C | 2C | 11.9 | 8.3 | 5.7 |
| B | 5C | 5C | 5C | 5C | 4C | 3C | 2C (14.2) |

*Tested in the form of its dihydrochloride.

TABLE II
Effects of 5-[(7-chloro-4-quinolinyl)amino]-3-[(alkylamino)methyl]-[1,1′-biphenyl]-2-ols and N-oxides against trophozoite-induced *P. Berghei* in mice

| X | Y | $NR_1R_2$ | Z | MST after single s.c. dose, mg/kg | | | | | | | |
|---|---|---|---|---|---|---|---|---|---|---|---|
| | | | | 640 | 320 | 160 | 80 | 40 | 20 | 10 | 5 |
| 4—Cl | H | $N(Et)_2$ | 0 | 1C(11.9) | 2C(11.2) | 4C(11.9) | 5C | 5C | 1C(33.7) | 1C(30.2) | 20.7 |
| 4—Cl | H | $N(Et)_2$ | 1 | 5C | 5C | 5C | 5C | 4C(37.9) | 3C(20.9) | 2C(14.2) | |
| 4—Cl | H | $NHC(CH_3)_3$ | 0 | 16.7 | 1C(16.4) | 2C(15.2) | 3C(14.9) | 4C(19.9) | 3C(14.4) | 2C(14.9) | 1C(11.4) |
| 4—Cl | H | $NHC(CH_3)_3$ | 1 | 4C(13.9) | 4C(13.9) | 3C(13.4) | 2C(20.6) | 4C(24.9) | 4C(28.9) | 1C(26.3) | 1C(10.3) |
| 3—Cl | H | $N(Et)_2$ | 0 | 4C(21.9) | 5C | 4C(31.9) | 2C(31.9) | 2C(25.2) | 15.7 | 15.5 | 3.9 |
| 3—Cl | H | $N(Et)_2$ | 1 | 3C(16.4) | 4C(21.9) | 5C | 2C(25.6) | 1C(25.9) | 16.5 | 9.7 | 2.1 |
| 3—$CF_3$ | H | $N(Et)_2$ | 0 | 4C(29.0) | 3C(21.9) | 4C(21.9) | 2C(28.2) | 3C(37.5) | 13.1 | 1C(15.2) | 13.5 |
| 3—$CF_3$ | H | $N(Et)_2$ | 1 | 4C(27.9) | 1C(24.9) | 21.1 | 11.7 | 8.1 | 6.9 | | |
| 2—Cl | H | $N(Et)_2$ | 0 | 5C | 4C(41.9) | 4C(41.9) | 5C | 3C(40.9) | 2C(17.2) | | |
| 2—Cl | H | $N(Et)_2$ | 1 | 5C | 2C(37.9) | 2C(18.9) | 2C(14.9) | 9.4 | 6.2 | 2.6 | 6.4 |

TABLE II (Continued)

| X | Y | $NR_1R_2$ | Z | 640 | 320 | 160 | 80 | 40 | 20 | 10 | 5 |
|---|---|---|---|---|---|---|---|---|---|---|---|
| 2—OMe | H | $N(Et)_2$ | 0 | 5C | 2C(37.9) | 2C(18.9) | 2C(14.9) | 10.3 | 8.9 | | |
| 2—OMe | H | $N(Et)_2$ | 1 | 3C(37.4) | 1C(36.2) | 2C(8.9) | 8.7 | 6.9 | 0.7 | | |
| 3—Cl | 4—Cl | $N(Et)_2$ | 0 | 3C(10.9) | 5C | 5C | 4C(33.4) | 2C(28.9) | 15.7 | 0.9 | 0.3 |
| 3—Cl | 4—Cl | $N(Et)_2$ | 1 | 5C | 5C | 2C(29.1) | 2C(25.7) | 1C(21.2) | 10.5 | 5.9 | 0.7 |
| 3—Cl | 4—Cl | 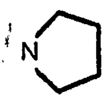 | 0 | 3C(33.9) | 3C(25.9) | 4C(27.4) | 1C(30.2) | 20.0 | 8.3 | 0.1 | −0.1 |
| 3—Cl | 4—Cl | 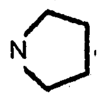 | 1 | 5C | 3C(24.4) | 2C(25.4) | 8.8 | 7.9 | 2.3 | 0.7 | |
| 3—Cl | 4—Cl | $NHC(CH_3)_3$ | 0 | 4C(16.4) | 3C(14.4) | 4C(13.4) | 4C(30.4) | 3C(27.4) | 1C(10.5) | 9.7 | 6.1 |
| 3—Cl | 4—Cl | $NHC(CH_3)_3$ | 1 | 5C | 3C(25.9) | 1C(25.9) | 1C(23.2) | 12.0 | 5.9 | 2.9 | 0.5 |
| 4—$OCH_3$ | H | $N(Et)_2$ | 0 | 5C | 3C(28.9) | 2C(16.1) | 1C(12.4) | 10.0 | 4.3 | 2.9 | 0.1 |
| 4—$OCH_3$ | H | $N(Et)_2$ | 1 | 4C(27.4) | | 7.3 | | 3.5 | 3.7 | 1.1 | 0.1 |
| 4—Cl | H | $NHCH(CH_3)C_2H_5$ | 0 | 5C(14.2) | 1C(12.5) | 3C(11.9) | 3C(14.0) | 4C(15.0) | 3C(21.0) | 23.6 | 11.0 |

TABLE II (Continued)

| X | Y | $NR_1R_2$ | Z | 640 | 320 | 160 | 80 | 40 | 20 | 10 | 5 |
|---|---|---|---|---|---|---|---|---|---|---|---|
| 4—Cl | H | $NHCH(CH_3)C_2H_5$ | 1 | 3C(11.6) | 3C(17.1) | 4C(14.6) | 3C(23.6) | 3C(24.3) | 2C(13.8) | 7.2 | |
| 4—Cl | H | $NHCH_2CH(CH_3)_2$ | 0 | 3C(14.1) | 2C(11.3) | 4C(7.6) | 5C | 4C(37.6) | 2C(19.6) | 14.8 | 5.2 |
| 4—$CF_3$ | H | $N(Et)_2$ | 0 | 12.1 | | 1C(12.2) | | 2C(17.5) | | | |
| 4—$CF_3$ | H | $NHC(CH_3)_3$ | 0 | 13.7 | | 1C(14.5) | | 2C(16.8) | | | |
| 4—$CF_3$ | H | $N(C_2H_5)_2$ | 1 | 1C(12.3) | | 2C(20.5) | | 2C(25.2) | | | |
| 4—Cl | H | $N(C_3H_7)_2$ | 0 | 3C)13.3) | 4C | 2C(26.6) | 3C(27.6) | 1C(22.2) | 12.4 | 6.8 | |
| 4—Cl | H | $N(CH_3)_2$ | 0 | 1C(18.7) | 1C(14.4) | 5C | 4C(45.4) | 1C(29.7) | 1C(24.7) | 20.4 | (14.2) |
| 4—Cl | H | $N(CH_3)_2$ | 1 | 1C(20.7) | 3C(17.9) | 5C | 3C(21.9) | 3C(21.4) | 2C(19.7) | 1C(17.8) | 17.6 |
| 4—Cl | H | $N(C_4H_9)_2$ | 0 | 3C(34.4) | 24.0 | 17.0 | 12.6 | 8.2 | 4.6 | | |
| 4—Cl | H | $N(C_4H_9)_2$ | 1 | 1C(31.7) | 21.2 | 12.2 | 8.0 | 2.2 | | | |
| 4—F | H | $N(Et)_2$ | 0 | 14.8 | | 12.8 | | 2C(13.4) | | | |
| 4—OH | H | $N(Et)_2$ | 0 | 1C(4.1) | 2C(3.6) | 6.8 | 4.8 | | | | |
| 4—F | H | $N(Et)_2$ | 1 | 15.0 | 4C(8.6) | 5C | 4C(23.6) | 2C(23.6) | 21.4 | | |
| 4—F | H | $NHC(CH_3)_3$ | 0 | 14.4 | 16.6 | 1C(11.9) | 1C(12.4) | 4C(10.6) | 4C(17.6) | | |
| 4—Cl | H | $NHC_6H_{11}$ | 0 | 4C(17.6) | 4C | 4C(20.6) | 3C(23.6) | 1C(21.4) | 1C(13.1) | | |

Starting Materials

A. 5-Amino-4′-chloro-3-[(diethylamino)methyl]-1,1′-biphenyl-2-ol, dihydrochloride may be prepared as follows:

A solution of 6.5 g of N-[4′-chloro-6-hydroxy-[1,1′-biphenyl]-3-yl]acetamide, 2.0 ml of 37% aqueous formaldehyde and 2.6 ml of diethylamine in 20 ml of ethanol is heated under reflux for 7 hr. After the second, fourth and sixth hours, additional portions of one ml of formaldehyde and one ml of diethylamine are added. The mixture is allowed to remain overnight, the solvent is removed *in vacuo*, and the residue is dissolved in ether. The solution is washed with water and with saturated sodium chloride solution, dried over sodium sulfate and evaporated to dryness *in vacuo*. The residue is dissolved in ethyl acetate and chromatographed over 200 g of alumina (Alcoa F—20) with ethyl acetate to provide N-[4′-chloro-5-[(diethylamino)methyl]-6-hydroxy-[1,1′-biphenyl]-3-yl]acetamide. A mixture of this material (8.3 g) in 15 ml of water and 17 ml of concentrated hydrochloric acid is heated under reflux for 2 hr, allowed to cool overnight, and evaporated *in vacuo* to provide 5-amino-4′-chloro-3-[(diethylamino)methyl]-1,1′-biphenyl-2-ol,dihydrochloride. (Table III, compound a.)

Additional starting materials, which may be prepared by the above route, are shown in Table III.

TABLE III

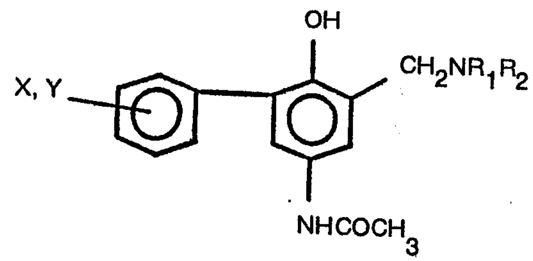

| No. | X | Y | $NR_1R_2$ | mp°C | Recrystallization Solvent |
|---|---|---|---|---|---|
| a) | 4—Cl | H | $N(C_2H_5)_2$ | — | — |
| b) | 4—Cl | H | $NHC(CH_3)_3$ $(H_3PO_4)$ | 241—3(dec) | — |
| c) | 4—Cl | H | $NHCHC_2H_5$ with $CH_3$ on CH | oil | — |
| d) | 4—Cl | H | $NHCH_2CH(CH_3)_2$ | gum | — |
| e) | 4—Cl | H | $N(C_3H_7)_2$ | oil | — |
| f) | 4—Cl | H | $N(CH_3)_2$ | glass | — |
| g) | 4—Cl | H | $N(C_4H_9)_2$ | oil | — |
| h) | 4—Cl | H | $NH(C_6H_{11})$ | | |
| i) | 3—Cl | H | $N(C_2H_5)_2$ | oil | Chromatography |
| j) | 3—$CF_3$ | H | $N(C_2H_5)_2$ | 115—8 | $CH_3CHOHCH_3$—$H_2O$ |
| k) | 2—Cl | H | $N(C_2H_5)_2$ | gum | Chromatography |

| No. | X | Y | $NR_1R_2$ | mp°C |
|---|---|---|---|---|
| l) | 4—Cl | 3—Cl | $N(C_2H_5)_2$ | 75—85 |
| m) | 4—Cl | 3—Cl | $N(CH_2)_4$ | gum |
| n) | 4—Cl | 3—Cl | $NHC(CH_3)_3$ | 164—5 |
| o) | 4—$CF_3$ | H | $N(C_2H_5)_2$ | glass |
| p) | 4—$CF_3$ | H | $NHC(CH_3)_3$ | oil |
| q) | 4—F | H | $N(C_2H_5)_2$ | oil |
| r) | 4—OH | H | $N(C_2H_5)_2$ | oil |
| s) | 2—$CF_3$ | H | $N(C_2H_5)_2$ | glass |
| t) | 3—F | H | $N(C_2H_5)_2$ | oil |
| u) | 4—$CH_3S$ | H | $N(C_2H_5)_2$ | oil |
| v) | 2—F | H | $N(C_2H_5)_2$ | oil |
| w) | 4—F | H | $NHC(CH_3)_3$ | oil |

B. N-[4′-Chloro-6-hydroxy-[1,1′-biphenyl]-3-yl]acetamide.

A solution of 7.0 g of 5-amino-4′-chloro-1,1′-biphenyl]-2-ol and 3 ml of acetic anhydride in 400 ml of toluene is treated with charcoal, heated to boiling, filtered and cooled. The solid is recrystallized from toluene to give N-[4′-chloro-6-hydroxy-[1,1′-biphenyl]-3-yl]acetamide, mp 135°C. (Table IV, compound a.)

Additional starting materials, which may be prepared by the above route, are shown in Table IV.

TABLE IV

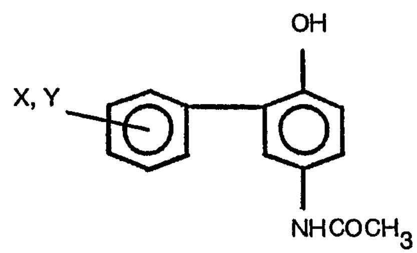

| No. | X | Y | mp°C | Recrystallization Solvent |
|---|---|---|---|---|
| a) | 4—Cl | H | 135 | Toluene |
| b) | 3—Cl | H | 152—4 | Toluene[1] |
| c) | 3—$CF_3$ | H | 145—7 | Toluene |
| d) | 2—Cl | H | 111—7 | Toluene[1] |
| e) | 4—Cl | 3—Cl | 187—9 | Toluene[1] |
| f) | 4—$CF_3$ | H | 186—8 | Toluene-$CH_3CHOHCH_3$ |
| g) | 4—F | H | glass | — |
| h) | 4—$CH_3O$—<br>$(CH_2)_2OCH_2O$ | H<br>H | glass | — |
| i) | 2—$CF_3$ | H | gum | — |
| j) | 3—F | H | — | — |
| k) | 4—$CH_3S$ | H | 145—7 | $CH_3CN$ |
| l) | 2—F | H | — | — |

[1] Triturated with the solvent.

C. 5-Amino-4′-chloro-[1,1′-biphenyl]-2-ol.

A mixture of 99.5 g of 2-iodoanisole, 202 g of 1-chloro-4-iodobenzene and 400 g of copper powder is stirred in an oil bath at 220—240° for 6 hr, allowed to cool overnight, and extracted with chloroform. The extract is dried over anhydrous sodium sulfate and concentrated to dryness *in vacuo.* The residue is taken up in 1 liter of hot pentane, filtered, and applied on a column of 800 g of silica gel equilibrated with hexane. Elution with hexane containing 2% ethyl ether provided 4′-chloro-2-methoxy-1,1′-biphenyl; mp 50—52°C after recrystallization from hexane.

A mixture of 31.5 g of 4′-chloro-2-methoxy-1,1′-biphenyl and 126 ml of 48% hydrobromic acid in 600 ml of glacial acetic acid is heated under reflux for 18 hr, allowed to cool, poured into 1 liter of water and extracted with ether. The extract is washed with water, dried over sodium sulfate and concentrated. The residue is recrystallized from hexane to give 4′-chloro-[1,1′-biphenyl]-2-ol, mp 49.5—52°C.

A solution of 4 g of 4′-chloro-[1,1′-biphenyl]-2-ol in 63 ml of glacial acetic acid and 40 ml of water is chilled to 5°C and treated dropwise with a solution of 1.5 g of sodium nitrite in 7 ml of water. The mixture is stirred several hours at 5°C and filtered. Recrystallization first from 33% acetic acid and then from toluene gives 2-(4-chlorophenyl)-2,5-cyclohexadiene-1,4-dione,4-oxime, mp 182—184°C (dec).

To a stirred suspension of 7.8 g of 2-(4-chlorophenyl)-2,5-cyclohexadiene-1,4-dione,4-oxime in 170 ml of 1*N* sodium carbonate is added in portions 23 g of sodium dithionite. The mixture is stirred for 5 hrs, filtered, washed with water and recrystallized from toluene to give 5-amino-4′-chloro-[1,1′-biphenyl]-2-ol, mp 189—191°C.

D. N-[2′-Chloro-6-hydroxy[1,1′-biphenyl]-3-yl]acetamide.

A solution of 19.9 g of 2′-chloro-5-nitro[1,1′-biphenyl]-2-ol in 200 ml of methanol is hydrogenated over 0.7 g of Raney nickel at an initial pressure of 52 psi at room temperature for 23 hrs. An additional gram of catalyst is added and hydrogenation continued for another 8 hrs. The mixture is filtered into 8.2 ml of acetic anhydride, warmed on the steam bath for 20 minutes and concentrated to dryness *in vacuo*. Chromatography of the residue over 190 g of silica gel with ethyl acetate affords a gum which is triturated with toluene to give N-[2′-chloro-6-hydroxy[1,1′-biphenyl]-3-yl]acetamide, mp 111—117°C.

A mixture of 18.9 g of sodium nitromalonaldehyde in 210 ml of water and 76 ml of 10% sodium hydroxide is combined with a solution of 25 g of *o*-chlorophenylacetone in 265 ml of ethanol. The mixture is allowed to remain at room temperature for 20 hrs, concentrated *in vacuo* to remove the ethanol, extracted with ether, and the aqueous layer is acidified with 10*N* hydrochloric acid. The resulting oil is extracted with ether. The extract is washed, dried and concentrated *in vacuo* to dryness. Recrystallization from toluene gives 2′-chloro-5-nitro-[1,1′-biphenyl]-2-ol, mp 155—157°C.

Additional starting materials, which may be prepared by the same route are shown in Table V.

TABLE V

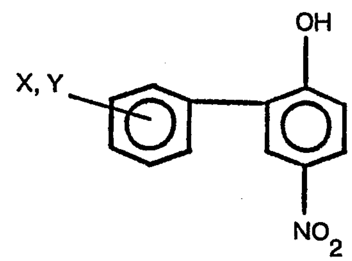

| No. | X | Y | mp°C | Recrystallization Solvent | No. | X | Y | mp°C | Recrystallization Solvent |
|---|---|---|---|---|---|---|---|---|---|
| a) | 3—Cl | H | 180—2 | Toluene $CH_3CH_2OH$ | g) | 3—F | H | 143—6 | Toluene |
| b) | 3—$CF_3$ | H | 185—7 | Toluene $CH_3CHOHCH_3$ | h) | 4—$CH_3S$ | H | 162—5 | Toluene |
| c) | 4—Cl | 3—Cl | 243—5 | $CH_3CHOHCH_3$ | i) | 2—F | H | — | — |
| d) | 4—$CF_3$ | H | 185—8 | Toluene | | | | | |
| e) | 4—F | H | 182—4(dec) | Ether-hexane | | | | | |
| f) | 2—$CF_3$ | H | 125—7 | $CH_2Cl_2$-hexane | | | | | |

Example 1

4′-chloro-5-[(7-chloro-4-quinolinyl)amino-3-[(diethylamino)methyl]-[1,1′-biphenyl]-2-ol,1-oxide.

A mixture of N-[4′-chloro-5-[(diethylamino)methyl]-6-hydroxy-[1,1′-biphenyl]-3-yl]acetamide (8.3 g) in 15 ml of water and 17 ml of concentrated hydrochloric acid is heated under reflux for 2 hrs, allowed to cool overnight, and evaporated *in vacuo* to provide 5-amino-4′-chloro-3-[(diethylamino)methyl]-1,1′-biphenyl-2-ol,dihydrochloride.

A mixture of 3.3 g of 4,7-dichloroquinoline,1-oxide and 5.7 g of 5-amino-4′-chloro-3-[(diethylamino)methyl]-1,1′-biphenyl-2-ol,dihydrochloride in 130 ml of ethanol is heated under reflux for 1.5 hrs, cooled and concentrated to dryness *in vacuo*. The dark residue is taken up in a mixture of 10% sodium carbonate and chloroform, and the resulting gold precipitate is collected and washed with 2-propanol to afford 4.5 g of crude product. Chromatography on silica with 10% methanol in ethyl acetate followed by recrystallization from ethanol provides 4′-chloro-5-[(7-chloro-4-quinolinyl)amino]-3-[(diethylamino]methyl]-[1,1′-biphenyl]-2-ol,1-oxide, mp 210—213°C (dec).

Example 2

4′-chloro-5-[(7-chloro-4-quinolinyl)amino]-3-[(diethylamino)methyl][1,1′-biphenyl]-2-ol.

A mixture of 8.3 g of N-[4′-chloro-5-[(diethylamino)methyl]-6-hydroxy-[1,1′-biphenyl]-3-yl]acetamide in 15 ml of water and 17 ml of concentrated hydrochloric acid is heated under reflux for 2 hrs, allowed to cool overnight, and evaporated *in vacuo*. The residue and 4.8 g of 4,7-dichloroquinoline in 40 ml of ethanol is heated under reflux for 1.5 hrs, allowed to cool and poured with stirring into 700 ml of 2*N* ammonium hydroxide. The precipitate is collected, washed with water and dissolved in chloroform. The extract is washed with dilute sodium hydroxide and with water, dried over anhydrous potassium carbonate, filtered and evaporated *in vacuo*. The residue is recrystallized from an acetonitrile-toluene mixture (800 ml: 300 ml) to provide 4′-chloro-5-[(7-chloro-4-quinolinyl)amino]-3-[(diethylamino)methyl][1,1′-biphenyl]-2-ol, mp 229—232°C.

Example 3

4′-chloro-5-[(7-chloro-4-quinolinyl)amino]-3-[[(1,1-dimethylethyl)amino]methyl][1,1′-biphenyl]-2-ol.

A mixture of 1.8 g of *N*-[4′-chloro-5-[[(1,1-dimethylethyl)amino]methyl]-6-hydroxy[1,1′-biphenyl]-3-yl]*acetamide* and 12 ml of 6*N*-hydrochloric acid is heated under reflux for 1.5 hrs and concentrated to dryness *in vacuo* to afford 5-amino-4′-chloro-3-[[(1,1-dimethylethyl)amino]-methyl][1,1′-biphenyl]-2-ol which is combined with 1 g of 4,7-dichloroquinoline and 15 ml of ethanol. The mixture is heated under reflux for 1 hr, allowed to cool, and poured into 250 ml of water containing 10 ml of concentrated ammonium hydroxide. The resulting precipitate is collected and dissolved in chloroform; the solution is washed with 1% sodium hydroxide solution and with water, dried over sodium sulfate, and evaporated *in vacuo* to dryness. Recrystallization from acetonitrile gives 4′-chloro-5-[(7-chloro-4-quinolinyl)amino]-3-[[(1,1-dimethylethyl)amino]methyl][1,1′-biphenyl]-2-ol, mp 228°C (dec).

Example 4

2′-chloro-5-[(7-chloro-4-quinolinyl)amino]-3-[(diethylamino)methyl][1,1′-biphenyl]-2-ol.

A mixture of 2.5 g of N-[2′-chloro-5-[(diethylamino)methyl]-6-hydroxy-[1,1′-biphenyl]-3-yl]acetamide (solvated with 0.2 mole of ethyl acetate) in 15 ml of 6*N* hydrochloric acid is heated under reflux for 1 hr, diluted with ethanol, concentrated *in vacuo*, combined with additional ethanol and concentrated again. A mixture of the residue and 1.4 g of 4,7-dichloroquinoline in 50 ml of ethanol is heated under reflux for 2 hrs, allowed to cool, and made basic with ammonium hydroxide. The precipitate is collected and dissolved in a mixture of dichloromethane and 5% aqueous ammonium hydroxide. The organic layer is washed with water, dried and concentrated to dryness *in vacuo*. Recrystallization from toluene provides 2′-chloro-5-[(7-chloro-4-quinolinyl)amino]-3-[(diethylamino)-methyl][1,1′-biphenyl]-2-ol, mp 231—234°C (dec).

TABLE VI
Physical Data for 5-[(7-Chloro-4-quinolinyl)amino]-3-[(alkylamino)methyl][1,1′-biphenyl]-2-ols and N-oxides[1]

| X | Y | $NR_1R_2$ | Z | mp°C | Recrystallization Solvent |
|---|---|---|---|---|---|
| 4—Cl | H | $N(Et)_2$ | 0 | 229—232 | MeOH—Toluene |
| 4—Cl | H | $N(Et)_2$ | 1 | 210—213 | EtOH |
| 4—Cl | H | $NHC(CH_3)_3$ | 0 | 228(dec) | MeCN |
| 4—Cl | H | $NHC(CH_3)_3$ | 1 | 196(dec) | MeCN |
| 3—Cl | H | $N(Et)_2$ | 0 | 238—240(dec) | 2—Butanone |
| 3—Cl | H | $N(Et)_2$ | 1 | 224—225(dec) | MeOH |
| 3—$CF_3$ | H | $N(Et)_2$ | 0 | 234—236(dec) | EtOH |
| 3—$CF_3$ | H | $N(Et)_2$ | 1 | 227—229(dec) | MeOH |
| 2—Cl | H | $N(Et)_2$ | 0 | 231—234(dec) | Toluene |
| 2—Cl | H | $N(Et)_2$ | 1 | 223—225(dec) | 2—PrOH(trit.) |
| 2—$OCH_3$ | H | $N(Et)_2$ | 0 | 130—133 (196—198) | 2—PrOH—$H_2O$ |
| 2—$OCH_3$ | H | $N(Et)_2$ | 1 | 220—222(dec) | 2—PrOH(trit.) |
| 3—Cl | 4—Cl | $N(Et)_2$ | 0 | 242—244(dec) | 2—PrOH—Toluene |
| 3—Cl | 4—Cl | $N(Et)_2$ | 1 | 186—190(dec) | 2—PrOH(trit.) |
| 3—Cl | 4—Cl | $N(CH_2)_4$ | 0 | 287—288(dec) | EtOH |
| 3—Cl | 4—Cl | $N(CH_2)_4$ | 1 | 245—246(dec) | DMF |
| 3—Cl | 4—Cl | $NHC(CH_3)_3$ | 0 | 220—222(dec) | Toluene |
| 3—Cl | 4—Cl | $NHC(CH_3)_3$ | 1 | 200—202(dec) | EtAc—$CH_2Cl_2$ |
| 4—$OCH_3$ | H | $N(Et)_2$ | 0 | 206—209 | MeCN |
| 4—$OCH_3$ | H | $N(Et)_2$ | 1 | 200—201(dec) | MeCN—2—PrOH |
| 4—Cl | H | $NHCH(CH_3)C_2H_5$ | 0 | 285—287(dec) | EtOH |
| 4—Cl | H | $NHCH(CH_3)C_2H_5$ | 1 | 193—195(dec) | MeCN—2—PrOH |
| 4—Cl | H | $NHCH_2CH(CH_3)_2$ | 0(·2HCl) | 277—280(dec) | EtOH |
| 4—$CF_3$ | H | $N(Et)_2$ | 0 | 227—229(dec) | MeCN |
| 4—$CF_3$ | H | $NHC(CH_3)_3$ | 0(·2HCl) | 292—294(dec) | EtOH |
| 4—$CF_3$ | H | $N(C_2H_5)_2$ | 1 | 169—172(dec) | — |
| 4—Cl | H | $N(C_3H_7)_2$ | 0 | 220—222 | DMF—MeOH |

TABLE VI (Continued)

| X | Y | $NR_1R_2$ | Z | mp°C | Recrystallization Solvent |
|---|---|---|---|---|---|
| 4—Cl | H | $N(CH_3)_2$ | 0 | 222—226 | EtOH |
| 4—Cl | H | $N(CH_3)_2$ | 1 | 160—175 | 2—PrOH |
| 4—Cl | H | $N(C_4H_9)_2$ | 0 | 166—170 | Cyclohexane |
| 4—Cl | H | $N(C_4H_9)_2$ | 1 | 160—166 | $CH_3CN$ |
| 4—F | H | $N(Et)_2$ | 0 | 225—228(dec) | EtOH |
| 4—OH | H | $N(Et)_2$ | 0 | 240—245(dec) | EtOH—$H_2O$ |
| 4—F | H | $N(Et)_2$ | 1 | 151—154(dec) | $CHCl_3$—EtAc |
| 4—F | H | $NHC(CH_3)_3$ | 0 | dec >154 | EtOH—$H_2O$ |
| 4—Cl | H | $NHC_6H_{11}$ | 0 | 106—120(dec) | Cyclohexane |

1) Compounds wherein Z is 1 are prepared by the process of Example 1 and compounds wherein Z is 0 are prepared by the process of Example 2.

**Claims for the Contracting States: BE CH LI DE FR GB IT LU NL SE**

1. A compound having the following general formula:

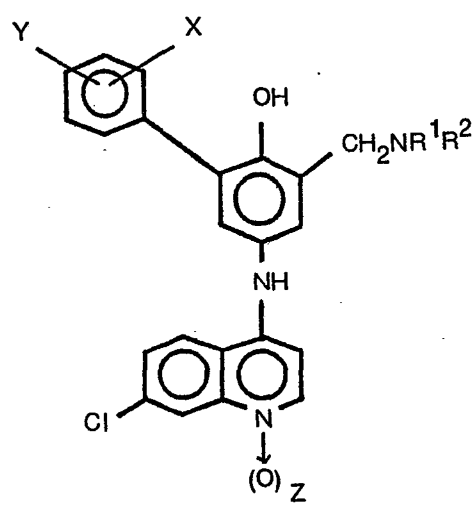

or a pharmaceutically acceptable salt thereof; wherein X is a hydrogen, fluorine, bromine or chlorine atom or a lower alkyl radical; Y is a chlorine, fluorine or bromine atom or a trifluoromethyl, lower alkoxy, cyano, hydroxy, nitro, lower alkylthio, amino, lower alkyl amino, di(lower alkyl) amino, pyrrolidino or piperidino radical; $R^1$ and $R^2$, which are the same or different, are each a hydrogen atom or a lower alkyl radical, or $R^1R^2N$ taken together is a pyrrolidino, piperidino or homopiperidino radical of which the heterocyclic ring is unsubstituted or substituted by from one to four lower alkyl radicals; Z is zero or one; a lower alkyl radical is an alkyl radical containing from one to six carbon atoms; and a lower alkoxy radical is an alkoxy radical containing from one to six carbon atoms.

2. A compound according to claim 1, wherein X is a hydrogen or chlorine atom, Y is a chlorine or fluorine atom, $R^1$ is a lower alkyl radical, $R^2$ is a hydrogen atom or a lower alkyl radical, and Z is zero or one.

3. A compound according to claim 1 or 2, wherein X is in the 3-position and Y is in the 2- or 4-position.

4. The compound 4′-chloro-5-[(7-chloro-4-quinolinyl)amino]-3-[(diethylamino)methyl][1,1′-biphenyl]-2-ol,1-oxide; or a salt thereof.

5. The compound 4′-chloro-5-[(7-chloro-4-quinolinyl)amino]-3-[(diethylamino)methyl]-[1,1′-biphenyl]-2-ol; or a salt thereof.

6. The compound 4′ - chloro - 5 - [(7 - chloro - 4 - quinolinyl)amino] - 3 - [[(1,1 - dimethyl - ethyl)amino]methyl][1,1′-biphenyl]-2-ol; or 4′-chloro-5-[(7-chloro-4-quinolinyl)amino]-3-[[(1,1-dimethylethyl)amino]methyl][1,1′-biphenyl]-2-ol,1-oxide; or a salt thereof.

7. The compound 2′-chloro-5-[(7-chloro-4-quinolinyl)amino]-3-[(diethylamino)methyl][1,1′-biphenyl]-2-ol; or a salt thereof.

8. The compound 3′,4′ - dichloro - 5 - [(7 - chloro - 4 - quinolinyl)amino] - 3 - [[(1,1 - dimethyl - ethyl)amino]methyl][1,1′-biphenyl]-2-ol; or a salt thereof.

9. A process for producing a compound according to Claim 1, which comprises coupling a compound having the following general formula:—

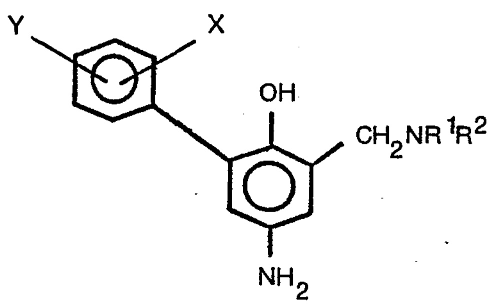

with a compound having the following general formula:—

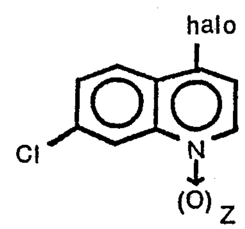

wherein $R^1$, $R^2$, X, Y and Z are as defined in Claim 1 and halo is chloro, bromo or iodo.

10. A pharmaceutical composition comprising a compound according to any one of Claims 1 to 8, and a pharmaceutical carrier therefor.

**Claims for the Contracting State: AT**

1. A process for producing a compound having the following general formula:—

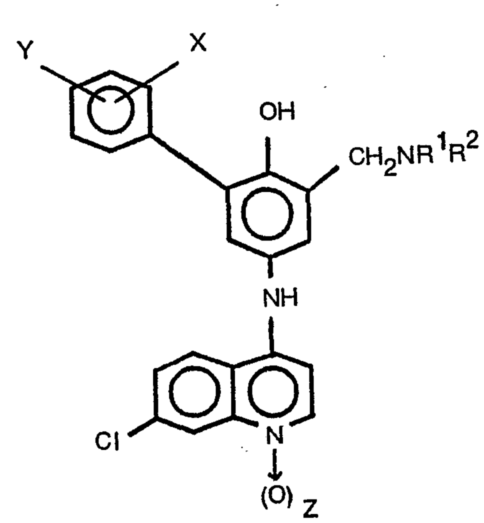

or a pharmaceutically acceptable salt thereof; wherein X is a hydrogen, fluorine, bromine or chlorine atom or a lower alkyl radical; Y is a chlorine, fluorine or bromine atom or a trifluoromethyl, lower alkoxy, cyano, hydroxy, nitro, lower alkylthio, amino, lower alkyl amino, di(lower alkyl) amino, pyrrolidino or piperidino radical; $R^1$ and $R^2$, which are the same or different, are each a hydrogen atom or a lower alkyl radical, or $R^1R^2N$ taken together is a pyrrolidino, piperidino or homopiperidino radical of which the heterocyclic ring is unsubstituted or substituted by from one to four lower alkyl radicals; Z is zero or one; a lower alkyl radical is an alkyl radical containing from one to six carbon atoms; and a lower alkoxy radical is an alkoxy radical containing from one to six carbon atoms; the process comprises coupling a compound having the following general formula:—

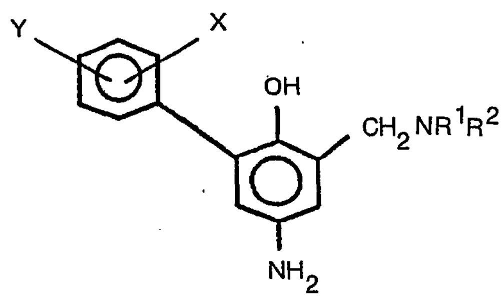

with a compound having the following general formula:—

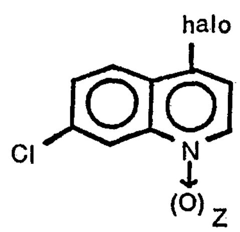

wherein $R^1$, $R^2$, X, Y and Z are as defined above and halo is chloro, bromo or iodo: and; where necessary, converting the produced compound to the desired salt.

2. A process according to Claim 1, wherein X is a hydrogen or chlorine atom, Y is a chlorine or fluorine atom, $R^1$ is a lower alkyl radical, $R^2$ is a hydrogen atom or a lower alkyl radical, and Z is zero or one.

3. A process according to Claim 1 or 2, wherein X is in the 3-position and Y is in the 2- or 4-position.

4. A process according to Claim 1, wherein the compound produced is 4′-chloro-5-[(7-chloro-4-quinolinyl)amino]-3-[(diethylamino)methyl]-[1,1′-biphenyl]- 2-ol,1-oxide.

5. A process according to Claim 1, wherein the compound produced is 4′-chloro-5-[(7-chloro-4-quinolinyl)amino]-3-[(diethylamino)methyl]-[1,1′-biphenyl]-2-ol.

6. A process according to Claim 1, wherein the compound produced is 4′-chloro-5-[(7-chloro-4-quinolinyl)amino]-3-[[(1,1-dimethylethyl)amino]methyl][1,1′-biphenyl]-2-ol.

7. A process according to Claim 1, wherein the compound produced is 4′-chloro-5-[(7-chloro-4-quinolinyl)amino]-3-[[(1,1-dimethylethyl)amino]methyl]-[1,1′-biphenyl]-2-ol,1-oxide.

8. A process according to Claim 1, wherein the compound produced is 2′-chloro-5-[(7-chloro-4-quinolinyl)amino]-3-[(diethylamino)methyl][1,1′-biphenyl]-2-ol.

9. A process according to Claim 1, wherein the compound produced is 3′,4′-dichloro-5-[(7-chloro-4-quinolinyl)amino]-3-[[(1,1-dimethylethyl)amino]methyl][1,1′-biphenyl]-2-ol.

**Patentansprüche für die Vertragsstaaten: BE CH LI DE FR GB IT LU NL SE**

1. Verbindung mit der folgenden allgemeinen Formel:

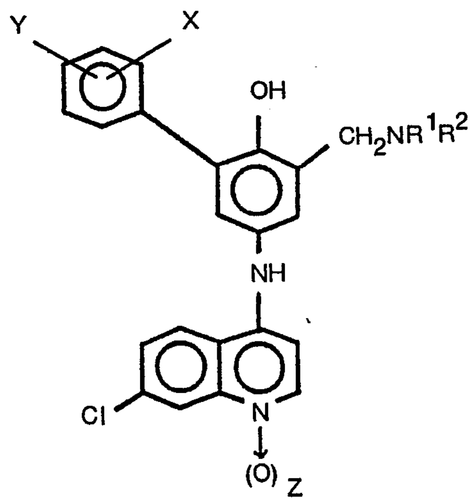

oder ein pharmazeutisch akzeptables Salz davon; worin X ein Wasserstoff-, Fluor-, Brom- oder Chloratom oder ein Niedrigalkylradikal bedeutet; Y ein Chlor-, Fluor- oder Bromatom oder ein Trifluormethyl, Niedrigalkoxy, Cyano, Hydroxy, Nitro, Niedrigalkylthio, Amino, Niedrigalkylamino, Di(niedrigalkyl)amino, Pyrrolidino oder Piperidino-Radikal bedeutet; $R^1$ und $R^2$, die gleich oder verschieden sind, jeweils ein Wasserstoffatom oder ein Niedrigalkylradikal bedeuten, oder $R^1R^2N$ zusammen ein Pyrrolidino, Piperidino oder Homopiperidino-Radikal bedeuten, von welchem der heterozyklische Ring unsubstituiert oder durch ein bis vier Niedrigalkylradikale substituiert ist; Z null oder eins ist; ein Niedrigalkylradikal ein Alkylradikal mit ein bis sechs Kohlenstoffatomen ist; und ein Niedrigalkoxyradikal ein Alkoxyradikal mit ein bis sechs Kohlenstoffatomen ist.

2. Verbindung nach Anspruch 1, worin X ein Wasserstoff- oder Chloratom bedeutet, Y ein Chlor- oder Fluor- atom bedeutet, $R^1$ ein Niedrigalkylradikal, $R^2$ ein Wasserstoffatom oder ein Niedrigalkylradikal bedeutet und Z null oder eins ist.

3. Verbindung nach Anspruch 1 oder 2, worin X in der 3-Position und Y in der 2- oder 4-Position ist.

4. Die Verbindung 4'-Chlor-5-[(7-chlor-4-chinolinyl)amino]-3-[(diäthylamino)methyl]-[1,1'-biphenyl]-2-ol,1-oxid; oder ein Salz derselben.

5. Die Verbindung 4'-Chlor-5-[(7-chlor-4-chinolinyl)amino]-3-[(diäthylamino)methyl]-[1,1'-biphenyl]-2-ol; oder ein Salz derselben.

6. Die Verbindung 4'-Chlor-5-[(7-chlor-4-chinolinyl)amino]-3-[[(1,1-dimethyläthyl)amino]methyl]-[1,1'-biphenyl]-2-ol; oder 4'-Chlor-5-[(7-chlor-4-chinolinyl)amino]-3-[[(1,1-dimethyläthyl)amino]methyl]-[1,1'-biphenyl]-2-ol, 1-oxia; oder eine Salz derselben.

7. Die Verbindung 2'-Chlor-5-[(7-chlor-4-chinolinyl)amino]-3-[(diäthylamino)methyl]-[1,1'-biphenyl]-2-ol; oder ein Salz derselben.

8. Die Verbindung 3',4'-Dichlor-5-[(7-chlor-4-chinolinyl)amino]-3-[[(1,1-dimethyläthyl)amino]methyl]-[1,1'-biphenyl]-2-ol; oder ein Salz derselben.

9. Verfahren zur Herstellung einer Verbindung nach Anspruch 1, dadurch gekennzeichnet, daß eine Verbindung der folgenden allgemeinen Formel:

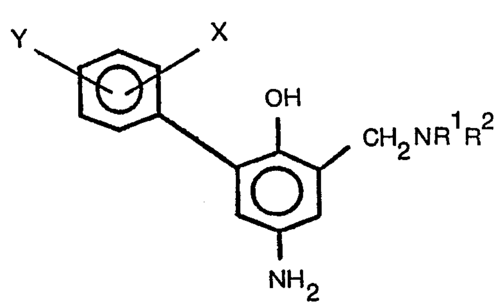

mit einer Verbindung der folgenden allgemeinen Formel:

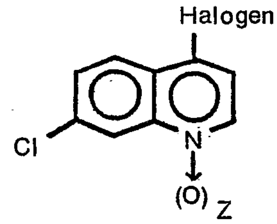

worin $R^1$, $R^2$, X, Y und Z die in Anspruch 1 angegebene Bedeutung haben und Halogen Chlor, Brom oder Jod bedeutet.

10. Pharmazeutische Zusammensetzung, umfassend eine Verbindung nach irgendeinem der Ansprüche 1 bis 8 und einen pharmazeutischen Träger dafür.

**Patentansprüche für den Vertragsstaat: AT**

1. Verfahren zur Herstellung einer Verbindung mit der folgenden allgemeinen Formel:

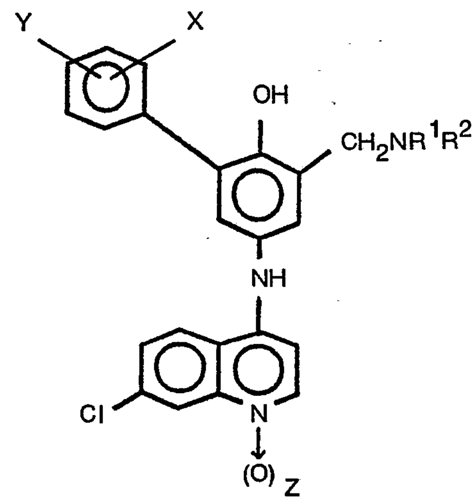

oder eines pharmazeutisch akzeptablen Salzes derselben; worin X ein Wasserstoff-, Fluor-, Brom- oder Chloratom oder ein Niedrigalkylradikal bedeutet; Y ein Chlor-, Fluor- oder Bromatom oder ein Trifluormethyl, Niedrigalkoxy, Cyano, Hydroxy, Nitro, Niedrigalkylthio, Amino, Niedrigalkylamino, Di(niedrigalkyl)-amino, Pyrrolidino oder Piperidino-Radikal bedeutet; $R^1$ und $R^2$, die gleich oder verschieden sind, jeweils ein Wasserstoffatom oder ein Niedrigalkylradikal, oder $R^1R^2N$ zusammen ein Pyrrolidino, Piperidino oder Homopiperidino-Radikal bedeuten, von welchem der heterozyklische Ring unsubstituiert oder durch ein bis vier Niedrigalkylradikale substituiert ist; Z null oder eins ist, ein Niedrigalkylradikal ein Alkylradikal mit ein bis sechs Kohlenstoffatomen ist; und ein Niedrigalkoxyradikal ein Alkoxyradikal mit ein bis sechs Kohlenstoffatomen ist; dadurch gekennzeichnet, daß eine Verbindung mit der allgemeinen Formel:

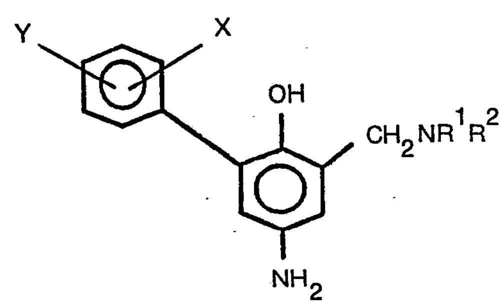

mit einer Verbindung der folgenden allgemeinen Formel:

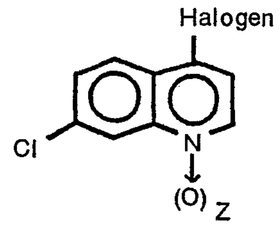

worin $R^1$, $R^2$, X, Y und Z die obige Bedeutung haben und Halogen Chlor, Brom oder Jod ist; gekoppelt wird; und, wo notwendig, die hergestellte Verbindung in das gewünschte Salz umgewandelt wird.

2. Verfahren nach Anspruch 1, worin X ein Wasserstoff- oder Chloratom, Y ein Chlor- oder Fluoratom, $R^1$ ein Niedrigalkylradikal, $R^2$ ein Wasserstoffatom oder ein Niedrigalkylradikal und Z null oder eins bedeuten.

3. Verfahren nach Anspruch 1 oder 2, dadurch gekennzeichnet, daß X in der 3-Position und Y in der 2- oder 4-Position ist.

4. Verfahren nach Anspruch 1, dadurch gekennzeichnet, daß die hergestellte Verbindung 4'-Chlor-5-[(7-chlor-4-chinolinyl)amino]-3-[(diäthylamino)methyl]-[1,1'-biphenyl]-2-ol,1-oxid ist.

5. Verfahren nach Anspruch 1, dadurch gekennzeichnet, daß die hergestellte Verbindung 4'-Chlor-5-[(7-chlor-4-chinolinyl)amino]-3-[(diäthylamino)methyl]-[1,1'-biphenyl]-2-ol ist.

6. Verfahren nach Anspruch 1, dadurch gekennzeichnet, daß die hergestellt Verbindung 4'-Chlor-5-[(7-chlor-4-chinolinyl)amino]-3-[[(1,1-dimethyläthyl)amino]methyl]-[1,1'-biphenyl]-2-ol ist.

7. Verfahren nach Anspruch 1, dadurch gekennzeichnet, daß die hergestellte Verbindung 4'-Chlor-5-[(7-chlor-4-chinolinyl)amino]-3-[[(1,1-dimethyläthyl)amino]methyl]-[1,1'-biphenyl]-2-ol,1-oxid ist.

8. Verfahren nach Anspruch 1, dadurch gekennzeichnet, daß die hergestellte Verbindung 2'-Chlor-5-[(7-chlor-4-chinolinyl)amino]-3-[(diäthylamino)methyl]-[1,1'-biphenyl]-2-ol ist.

9. Verfahren nach Anspruch 1, dadurch gekennzeichnet, daß die hergestellte Verbindung 3',4'-Dichlor-5-[(7-chlor-4-chinolinyl)amino]-3-[[(1,1-dimethyläthyl)amino]methyl]-[1,1'-biphenyl]-2-ol ist.

**Revendications pour les Etats contractants: BE CH LI DE FR GB IT LU NL SE**

1. Composé ayant la formule générale suivante:

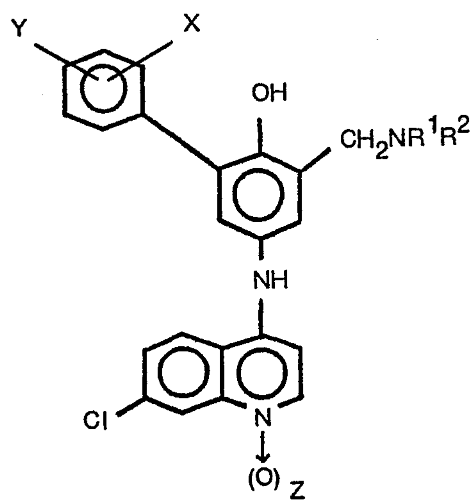

ou un de ses sels pharmaceutiquement acceptables, dans lesquels: X représente un atome d'hydrogène, de fluor, de brome ou de chlore, ou un radical alkyle inférieur; Y est un atome de chlore, de fluor ou de brome, ou un radical trifluorométhyle, alkoxy inférieur, cyano, hydroxy, nitro, alkylthio inférieur, amino, aminoalkyle inférieur, diaminoalkyle inférieur, pyrrolidino ou piperidino; $R^1$ et $R^2$ sont égaux ou différents l'un de l'autre et représentent chacun un atome d'hydrogène ou un radical alkyle inférieur; ou $R^1R^2N$ pris dans leur ensemble sont des radicaux pyrrolidino, piperidino ou homopiperidino dont le cycle hétérocyclique est non substitué ou substitué par 1 à 4 radicaux alkyles inférieurs; Z est égal à 0 ou à 1; un radical alkyles inférieur est un radical alkyle comprenant de 1 à 6 atomes de carbone, et un radical alkoxy inférieur est un radical alkoxy contenant de 1 à 6 atomes de carbone.

2. Composé suivant la revendication 1, dans lequel: X est un atome d'hydrogène ou de chlore, Y est un atome de chlore ou de fluor, $R^1$ est un radical alkyle inférieur, $R^2$ est un atome d'hydrogène ou un radical alkyle inférieur, et Z est égal à 0 ou à 1.

3. Composé suivant l'une des revendications 1 ou 2, dans lequel: X est en position 3, et Y est dans la position 2 ou la position 4.

4. Le composé 4'-chloro-5-{(7-chloro-4-quinolinyl)amino}-3-{(diéthylamino)méthyl)}-{1,1'-biphényl}-2-ol,1-oxyde; ou un des ses sels.

5. Le composé 4'-chloro-5-{(7-chloro-4-quinolinyl)amino}-3-{[diéthylamino)méthyl}-{1,1'-biphényl}-2-ol; ou un de ses sels.

6. Le composé 4'-chloro-5-{(7-chloro-4-quinolinyl)amino}-3-{{(1,1-diméthyléthyl)amino}méthyl}-{1,1'-biphényl}-2-ol; ou le 4'-chloro-5-{(7-chloro-4-quinolinyl)amino}-3-{{(1,1-diméthyléthyl)amino}méthyl}-{1,1'-biphényl}-2-ol,1-oxyde; ou un de leurs sels.

7. Le composé 2'-chloro-5-{(7-chloro-4-quinolinyl)amino}-3-{(diéthylamino)méthyl}-{1,1'-biphényl}-2-ol; ou un de ses sels.

8. Le composé 3',4'-dichloro-5-{(7-chloro-4-quinolinyl)amino}-3-{{(1,1-diméthyléthyl)amino}méthyl}-{1,1'-biphényl}-2-ol; ou un de ses sels.

9. Procédé de préparation d'un dérivé suivant la revendication 1, caractérisé en ce que l'on couple un composé ayant la formule générale:

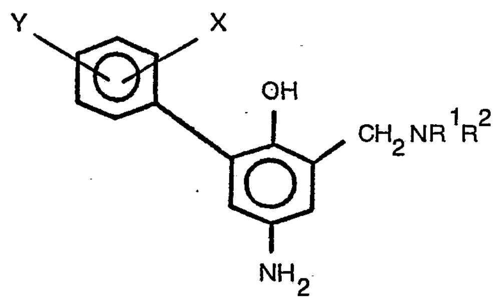

avec un composé ayant la formule générale suivante:

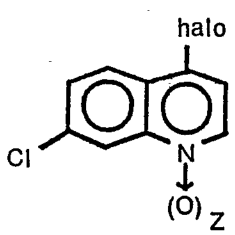

dans lesquels: $R^1$, $R^2$, X, Y et Z sont tels que décrits dans la revendication 1 et l'halogène est un atome de chlore, de brome ou d'iode.

10. Composition pharmaceutique comprenant un composé suivant l'une quelconque des revendications 1 à 8, et un véhicule pharmaceutique.

**Revendications pour l'Etat contractant: AT**

1. Procédé de préparation d'un composé ayant la formule générale suivant:

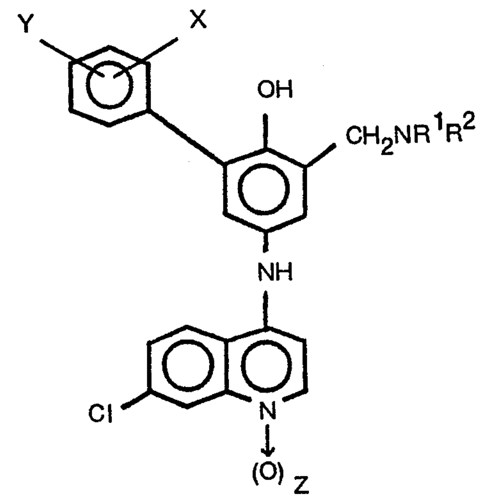

ou un sel de ce composé pharmaceutiquement acceptable, dans lequel: X est un atome d'hydrogène, de fluor, de brome ou de chlore, ou un radical alkyle inférieur; Y est un atome de chlore, de fluor ou de brome, ou un radical trifluorométhyle, alkoxy inférieur, cyano, hydroxy, nitro, alkylthio inférieur, amino, aminoalkyle inférieur, diaminoalkyle inférieur, pyrrolidino ou piperidino; $R^1$ et $R^2$ sont égaux ou différents l'un de l'autre et représentent chacun un atome d'hydrogène ou un radical alkyle inférieur; ou $R^1R^2N$ pris dans leur ensemble sont des radicaux pyrrolidino, piperidino ou homopiperidino dans lesquels le cycle hétérocyclique est non substitué ou substitué par 1 à 4 radicaux alkyles inférieurs; Z est égal à 0 ou à 1; un radical alkyle inférieur est un radical alkyle contenant de 1 à 6 atomes de carbone; et un radical alkoxy inférieur est un radical alkoxy contenant de 1 à 6 atomes de carbone. Ce procédé consistant à coupler un composè ayant la formule générale suivant:

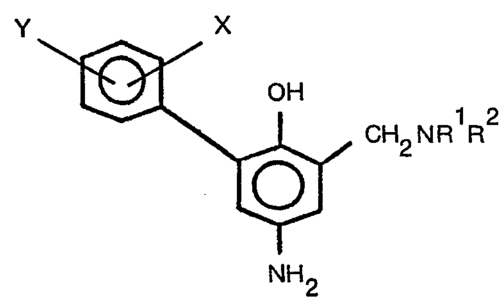

avec un composé ayant la formule générale suivante:

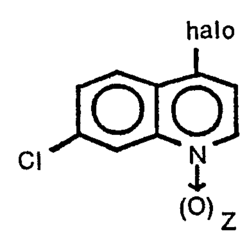

dans laquelle: $R^1$, $R^2$, X, Y et Z sont tels que définis ci-dessus, et le terme halo représente un atome de chlore, de brome ou d'iode, et si nécessaire, à transformer le composé produit en le sel souhaité.

2. Procédé suivant la revendication 1, dans lequel: X est un atome d'hydrogène ou de chlore; Y est un atome de chlore ou de fluor; $R^1$ est un radical alkyle inférieur; $R^2$ est un atome d'hydrogène ou un radical alkyle inférieur; et Z est égal à 0 ou à 1.

3. Procédé suivant l'une des revendications 1 ou 2, dans lequel: X est en position 3; et Y est en position 2 ou en position 4.

4. Procédé suivant la revendication 1, dans lequel le composé produit est le 4'-chloro-5-{(7-chloro-4-quinolinyl}-3-{(diéthylamino)méthyl}-{1,1'-biphényl}-2-ol,1-oxyde.

5. Procédé suivant la revendication 1, dans lequel le composé produit est le 4'-chloro-5-{(7-chloro-4-quinolinyl)amino}-3-{(diéthylamino)méthyl}-{1,1'-biphényl}-2-ol.

6. Procédé suivant la revendication 1, dans lequel le composé produit est le 4'-chloro-5-{(7-chloro-4-quinolinyl)amino}-3-{{(1,1-diméthyléthyl)amino}méthyl}-{1,1'-biphényl}-2-ol.

7. Procédé suivant la revendication 1, caractérisé en ce que le composé produit est le 4'-chloro-5-{(7-chloro-4-quinolinyl)amino}-3-{{(1,1-diméthyléthyl)amino}méthyl}-{1,1'-biphényl}-2-ol,1-oxyde.

8. Procédé suivant la revendication 1, caractérisé en ce que le composé produit est le 2'-chloro-5-{(7-chlor-4-quinolinyl)amino}-3-{(diéthylamino)méthyl}-{1,1'-biphényl}-2-ol.

9. Procédé suivant la revendication 1, caractérisé en ce que le composé produit est le 3',4'-dichloro-5-{(7-chloro-4-quinolinyl)amino}-3-{{(1,1-diméthyléthyl)amino}méthyl}-{1,1'-biphényl}-2-ol.